# FASCICULE DE BREVET EUROPEEN

(11) **EP 2 999 416 B1**
(45) Date de publication et mention de la délivrance du brevet: **15.03.2017**
(21) Numéro de dépôt: 14731693.9
(22) Date de dépôt: 20.05.2014
(51) Int. Cl.: A61B 17/04

(54) **INSTRUMENT DESTINÉ À ÊTRE UTILISÉ POUR MODIFIER LE VOLUME DE L'ESTOMAC D'UN PATIENT**
INSTRUMENT ZUR MODIFIZIERUNG DES VOLUMENS VON DES MAGENS EINES PATIENTEN
INSTRUMENT INTENDED TO BE USED TO MODIFY THE VOLUME OF THE STOMACH OF A PATIENT

(30) Priorité: 20.05.2013 FR 1354507
(43) Date de publication de la demande: 30.03.2016
(73) Titulaire: Cousin Biotech, 59117 Wervicq Sud (FR)
(72) Inventeur: LEBLANC, Jérome, F-59560 Comines (FR); SOLECKI, Gilles, F-59390 Lannoy (FR); BENCHETRIT, Salomon, F-69300 Calluire et Cuire (FR)
(74) Mandataire: Balesta, Pierre
(86) Numéro de dépôt international: PCT/FR2014/051171
(87) Numéro de publication internationale: WO 2014/188114

(56) Documents cités:
- WO-A2-01/49095
- US-A- 5 534 008
- US-A1- 2005 267 531
- US-A1- 2008 172 085
- US-A1- 2011 077 669
- US-B1- 6 547 797
- US-B1- 8 382 775

## Description

### Arrière-plan de l'invention

La présente invention concerne le domaine de la chirurgie bariatrique, et plus précisément les opérations visant à modifier le volume de l'estomac d'un patient souffrant d'obésité.

L'invention concerne plus spécifiquement un instrument destiné à être utilisé pour modifier le volume de l'estomac d'un patient.

Cet instrument est donc conçu pour réaliser une opération de gastroplastie par plicature gastrique et par voie laparoscopique. La plicature gastrique est une technique qui consiste à réaliser une restriction de l'estomac sans résection de ce dernier. Elle est généralement réalisée en laparoscopie par pliage puis suture de la paroi gastrique à l'aide d'instruments chirurgicaux.

Les procédures chirurgicales existantes qui permettent de réduire la taille de l'estomac sont nombreuses et diverses. Une des techniques connues consiste à réaliser une gastrectomie par agrafage vertical des parois antérieure et postérieure de l'estomac puis de réséquer la partie de l'estomac que l'on a isolée du flux gastrique. Cette technique dite de gastrectomie longitudinale en manchon est réalisée avec une agrafeuse linéaire qui réalise ensuite une découpe entre les deux lignes d'agrafes. Si cette technique permet d'enlever définitivement les deux tiers du volume gastrique en laissant un estomac modifié en forme de tube grâce à un agrafage vertical sur toute la hauteur de la grande courbure gastrique, elle présente l'inconvénient de faire apparaître des fistules qui peuvent provoquer des complications graves.

Une autre technique chirurgicale consiste à réaliser une gastroplastie dite verticale et calibrée par la pose d'une bande. Cette technique, décrite dans WO 2002/064041, consiste à agrafer l'estomac dans le prolongement de l'oesophage sur une longueur définie puis de poser une bande autour du prolongement réalisé pour éviter sa dilatation. Cette technique nécessite la mise en place d'un dispositif médical formé de deux plaques articulées par une charnière qui est implanté à long terme dans le corps du patient.

US 5 549 621 décrit un dispositif similaire. Ces deux dispositifs sont très compressifs et tendent à nécroser les tissus, en raison de quoi peuvent apparaître des fistules.

Ces techniques chirurgicales sont dites irréversibles dans la mesure où elles ne permettent pas un retour à une anatomie normale du système digestif si le patient ou le chirurgien le souhaitent.

Pour résoudre ces problèmes liés à l'apparition de fistules et à l'irréversibilité de l'opération, une autre technique de gastroplastie est apparue et permet d'invaginer l'estomac de façon réversible et sans risque d'apparition de fistules postopératoires.

US 2012/0330329 décrit une méthode de plicature laparoscopique de la grande courbure de l'estomac, réalisée par deux lignes d'agrafes. Cette technique opératoire a pour avantage de ne pas engendrer de fistules puisqu'il n'y a pas de résection partielle de l'estomac. Elle n'est cependant pas réversible car il serait très difficile, voire impossible, de retirer par laparoscopie la multitude d'agrafes parfois enfouies profondément dans les tissus gastriques.

US 2008/0319455 décrit une méthode et une instrumentation médicale qui permet la réalisation de l'invagination de l'estomac et la pose d'une rangée d'agrafes. Ce dispositif permet de s'affranchir du problème de l'apparition des fistules mais il n'est pas réversible du fait de la fixation du pli par des agrafes enfouies dans les tissus.

US2011/077669 décrit un guide de suture comprenant deux bras connectés par une charnière. Une bague coulissante autour des bras permet d'ouvrir et de fermer ces derniers. La bague est maintenue en position distale par un organe ressort afin de maintenir les bras en position fermée.

### Objet et résumé de l'invention

Un premier objet de la présente invention est de proposer un instrument destiné à être utilisé pour modifier le volume de l'estomac d'un patient qui permet de réaliser une plicature de l'estomac limitant sensiblement l'apparition de fistules tout en étant réversible.

L'invention atteint son but par le fait que l'instrument, présentant une extrémité proximale, comprend :
un corps ayant une extrémité distale;
un dispositif de serrage comportant des premier et second bras montés pivotants à l'extrémité distale du corps tout en étant pivotants l'un par rapport à l'autre, chacun des premier et second bras comportant une pluralité de guides pour le passage d'une aiguille à suture ;
un dispositif d'ouverture pour écarter les premier et second bras l'un de l'autre en les faisant pivoter l'un par rapport à l'autre, ledit dispositif d'ouverture comportant un organe ressort reliant les premier et second bras et tendant à les maintenir écartés l'un de l'autre ; et
un dispositif de fermeture pour rapprocher les premier et second bras l'un de l'autre en les faisant pivoter l'un par rapport à l'autre.

Le corps présente une forme longiligne permettant une utilisation en laparoscopie. Le corps présente une direction longitudinale.

Les premier et seconds bras forment une pince qui permet de rapprocher l'un de l'autre deux bourrelets de la paroi stomacale lorsque l'instrument est amené en position fermée. Les guides ménagés sur les bras ont pour fonction de guider l'aiguille de suture, ce qui permet de suturer aisément les bourrelets, par exemple par un surjet. Les guides sont avantageusement disposés à des intervalles réguliers afin d'assurer la pérennité de l'invagination et la reproductibilité d'un patient à l'autre.

Avantageusement, l'instrument présente une position ouverte dans laquelle les premier et second bras sont écartés l'un de l'autre grâce au dispositif d'ouverture, et une position fermée dans laquelle les premier et second bras sont amenés l'un vers l'autre grâce au dispositif de fermeture, et le dispositif de fermeture présente une position verrouillée dans laquelle l'instrument est maintenu en position fermée, et une position déverrouillée dans laquelle l'instrument est libre d'être amené en position ouverte par le dispositif d'ouverture.

De préférence, le dispositif de fermeture présente plusieurs positions verrouillées. En position verrouillée, le dispositif d'ouverture ne peut pas écarter les premier et second bras l'un de l'autre, tandis qu'en position déverrouillée, le dispositif d'ouverture est libre d'écarter les premier et second bras l'un de l'autre.

En d'autres termes, en position verrouillée, l'instrument est maintenu en position fermée, tandis qu'en position déverrouillée, l'instrument peut être amené en position ouverte grâce au dispositif d'ouverture.

Un intérêt est d'améliorer la sécurité de l'instrument en empêchant une ouverture intempestive des bras pendant la réalisation de la suture.

L'instrument présente donc une position ouverte dans laquelle les premier et second bras sont écartés l'un de l'autre, et une position fermée dans laquelle les bras sont amenés l'un vers l'autre pour pincer les tissus à suturer. De préférence, lorsque l'instrument est utilisé à vide, les premier et second bras sont agencés pour s'étendre parallèlement à la direction longitudinale du corps lorsque l'instrument est en position fermée. Bien évidemment, lors de l'utilisation, les bras gardent un certain écartement entre eux en raison de l'épaisseur des tissus se trouvant pincés entre les deux bras.

On comprend que le dispositif de fermeture permet d'amener l'instrument en position fermée, tandis que le dispositif d'ouverture permet d'amener l'instrument en position ouverte.

En fonctionnement, pour amener l'instrument en position fermée, le chirurgien tient le corps de l'instrument tout en actionnant le dispositif de fermeture dans un premier sens, par exemple en le poussant vers l'extrémité distale de l'instrument. Pour amener l'instrument en position ouverte, le chirurgien actionne le dispositif de fermeture dans un second sens, opposé au première sens, à la suite de quoi le dispositif d'ouverture écarte les premier et second bras l'un de l'autre.

De préférence, le dispositif de fermeture est actionné par le chirurgien, tandis que le dispositif d'ouverture agit de manière automatique. En d'autres termes, sans action du chirurgien, l'instrument tend à revenir en position ouverte lorsque le dispositif de fermeture est en position déverrouillée.

L'organe de ressort peut être un ressort cylindrique, une lame ressort ou tout autre dispositif élastique.

Avantageusement, chacun des premier et second bras présente une extrémité reliée de manière pivotante à l'extrémité distale du corps.

De préférence, les extrémités des premier et second bras reliées de manière pivotante à l'extrémité distale du corps sont montées pivotantes autour d'un même axe de rotation. L'instrument selon l'invention est donc simple et robuste dès lors qu'il existe une unique liaison pivot entre les premier bras, second bras et le corps.

Avantageusement, le dispositif de fermeture comporte un organe de fermeture actionnable coopérant avec les premier et second bras de sorte que l'actionnement de l'organe de fermeture par le chirurgien entraine le rapprochement des premier et second bras l'un de l'autre.

De préférence, l'instrument est amené en position fermée par un déplacement de l'organe de fermeture vers l'extrémité distale de l'instrument.

Encore de préférence, le corps présente une direction longitudinale, et l'organe de fermeture est monté sur le corps de manière coulissante parallèlement à ladite direction longitudinale.

Le corps présente à son extrémité proximale une portion de préhension permettant au chirurgien de maintenir fermement l'instrument pendant le déplacement de l'organe de fermeture vers l'extrémité distale de l'instrument.

Selon un mode de réalisation préférentiel, le dispositif de fermeture comporte un bras d'actionnement qui coopère avec un dispositif de guidage monté sur le corps permettant au bras d'actionnement de coulisser selon la direction longitudinale du corps ; l'organe de fermeture est disposé à une extrémité distale du bras d'actionnement et flanque les premier et second bras de telle sorte que le déplacement du bras d'actionnement vers l'extrémité distale de l'instrument entraîne le rapprochement des premier et second bras l'un de l'autre.

Le bras d'actionnement présente par exemple la forme d'une tige dont l'extrémité distale est fixée solidairement à l'organe de fermeture.

Selon une variante, le dispositif de guidage forme une glissière. Elle peut être simplement constituée d'une pièce comprenant deux nervures longitudinales parallèles à la direction longitudinale du corps. Dans ce cas, l'organe de fermeture enserre les deux bras de manière à assurer le maintien du bras d'actionnement par rapport au corps.

De préférence, l'extrémité proximale du bras d'actionnement est pourvue d'un organe de préhension pour faciliter la saisie et la manipulation dudit bras d'actionnement. Il pourra par exemple s'agir d'un retour lorsque le bras d'actionnement est une tige.

Lorsque l'instrument est en position ouverte, les premier et deuxième bras forment un « V » dont la pointe se trouve sensiblement à l'extrémité distale du corps. On comprend que l'organe de fermeture, flanquant les deux bras, coopère avec les faces latérales extérieures desdits bras. Aussi, un déplacement de l'organe de fermeture vers les extrémités distales des bras tend à refermer le « V » formé par les bras et, par voie de conséquence, a pour effet d'amener l'instrument en position fermée.

L'organe de fermeture comprend de préférence une pièce en forme de U présentant deux ailes en vis-à-vis qui sont disposées de part et d'autre de l'ensemble constitué des premier et second bras, grâce à quoi l'organe de fermeture flanque les deux bras.

De préférence, la distance entre les deux ailes est sensiblement égale ou légèrement supérieure à la distance entre les faces latérales extérieures des deux bras.

Pour limiter le déplacement de l'organe de fermeture vers l'extrémité distale de l'instrument, ce dernier comporte en outre au moins un organe de butée.

Lorsque l'organe de fermeture est au contact de l'organe de butée, le dispositif de fermeture est en position verrouillée. Dans cette position verrouillée, l'organe de fermeture, qui flanque les deux bras, permet d'empêcher leur écartement et, par voie de conséquence, assure le verrouillage. Bien évidemment, en utilisation, il est possible que l'organe de fermeture ne puisse pas être amené au contact des organes de butée en raison de la présence de tissu entre les bras. Pour autant le dispositif de fermeture sera en position verrouillée car il s'oppose à l'écartement des bras par l'organe ressort.

De surcroît, cet organe de butée permet d'empêcher l'organe de fermeture de se déplacer le long des portions des bras qui contiennent les guides.

Avantageusement, l'organe de butée fait saillie depuis l'un des premier et second bras. De préférence, l'organe de butée est disposé entre l'extrémité proximale du bras reliée au corps et les guides.

De préférence, chacun des premier et second bras porte un organe de butée.

Pour faciliter la prise des portions de parois, de l'estomac ou de bourrelets résultant des plicatures, au moins le premier bras comporte avantageusement une portion d'accroche s'étendant selon une face latérale intérieure du premier bras disposée en vis-à-vis d'une face latérale intérieure du second bras.

Cette portion d'accroche peut présenter une forme crénelée.

Selon une variante préférentielle, la portion d'accroche comprend une pluralité de picots s'étendant le long de la face latérale intérieure du premier bras.

De préférence, au moins le second bras comporte des orifices s'étendant selon la face latérale intérieure du second bras en vis-à-vis de la face latérale intérieure du premier bras, lesdits orifices étant destinés à recevoir les picots du premier bras lorsque les premier et second bras sont amenés l'un contre l'autre. Un intérêt de cette configuration de la portion d'accroche est que les bras peuvent être amenés en contact l'un contre l'autre, ou à tout le moins à proximité directe.

Encore de préférence, chacune des faces latérales intérieures des premier et second bras comporte une pluralité de picots et une pluralité d'orifices agencées pour coopérer avec les orifices et picots formés dans l'autre face latérale intérieure.

Selon un mode de réalisation avantageux, les guides débouchent transversalement de part et d'autre de chacun des premier et second bras.

En outre, les guides sont agencés de sorte que, en position fermée, les guides du premier bras sont disposés en vis-à-vis des guides du second bras de manière à former une succession de passages transversaux continus qui débouchent de part et d'autre des faces latérales extérieures des premier et second bras. Ce passage permet de guider le déplacement de l'aiguille selon toute la largeur de l'instrument.

Selon une mode de réalisation préférentiel, les premier et second bras présentent des faces supérieures, et dans lequel les guides sont des encoches débouchant dans les faces supérieures des premier et second bras.

Avantageusement, la section transversale des encoches présente une forme divergente s'ouvrant vers les faces supérieures. Un intérêt est de permettre le retrait aisé de l'instrument sans que le fil de suture, notamment mis en place par surjet, n'entrave le retrait de l'instrument.

Cette forme divergente est préférentiellement convexe de manière à ne pas s'opposer au retrait du fil hors de l'encoche.

L'invention porte en outre sur un système destiné à être utilisé pour modifier le volume de l'estomac d'un patient, comportant un instrument selon l'invention, une aiguille à suture, et au moins un fil de suture cranté dont les crans forment des harpons. Un intérêt de ce fil est de faciliter la réalisation du surjet en évitant que le fil ne se desserre pendant la suture. En pratique, une extrémité du fil comporte une boucle dans laquelle est introduite l'autre extrémité du fil. Les harpons ont pour fonction de n'autoriser le déplacement du fil dans la boucle que dans un seul sens. Selon les inventeurs, un tel fil n'a jamais été utilisé pour réaliser un surjet dans le cadre d'une opération de modification du volume de l'estomac.

De préférence, mais non exclusivement, le fil de suture cranté est du type V-Loc PBT Non-absorbable (marque déposée).

On décrit ci-après un procédé de modification du volume de l'estomac d'un patient, dans lequel :
- on saisit une portion du fundus de l'estomac ; et
- on réalise une plicature en amenant ladite portion du fundus vers le fond de l'estomac de manière à former deux bourrelets s'étendant sensiblement selon la grande courbure de l'estomac.

De manière avantageuse :
- on fournit un instrument selon l'invention ;
- on amène les deux bourrelets l'un contre l'autre à l'aide de l'instrument, en plaçant d'abord les premier et second bras de part et d'autre des bourrelets puis en actionnant l'instrument afin de rapprocher les premier et second bras l'un de l'autre ; puis
- on réalise un surjet à l'aide d'une aiguille à suture et d'un fil de suture afin de suturer ensemble les deux bourrelets, les guides de l'instrument permettant de guider le passage de l'aiguille à suture lors de la réalisation du surjet.

De préférence, la plicature est réalisée avec une pince de coelioscopie en tirant sur le fundus ou la grande tubérosité de l'estomac, à la suite de quoi l'invagination de l'estomac se forme de manière naturelle.

Tout en maintenant l'estomac dans cette position grâce à la pince de coelioscopie, on pince les deux bourrelets l'un contre l'autre à l'aide de l'instrument et on réalise le surjet.

De préférence, on réalise trois surjets pour ligaturer la plicature sur toute la longueur des bourrelets.

### Brève description des dessins

L'invention sera mieux comprise à la lecture de la description qui suit d'un mode de réalisation de l'invention donné à titre d'exemple non limitatif, en référence au dessin annexé, sur lequel :
- la figure **1** est une vue en perspective de l'instrument en position ouverte ;
- la figure **2** est une vue de détail de l'extrémité distale de l'instrument en position fermée ;
- la figure **3** est une vue éclatée de l'instrument selon l'invention ;
- la figure **3A** est une vue en coupe longitudinale d'une des encoches de l'un des bras ;
- la figure **4** illustre de manière schématique l'estomac d'un patient ;
- la figure **5** représente l'étape de plicature lors de laquelle se forment deux bourrelets;
- la figure **6** représente le début de l'étape de suture réalisée après que les bourrelets ont été amenés l'un contre l'autre grâce à l'instrument de la figure **1** ;
- la figure **7** illustre la fin de l'étape de suture ; et
- les figures **8** et **9** illustrent en détail le système selon l'invention et la réalisation du surjet à l'aide du fil cranté.

### Description détaillée de l'invention

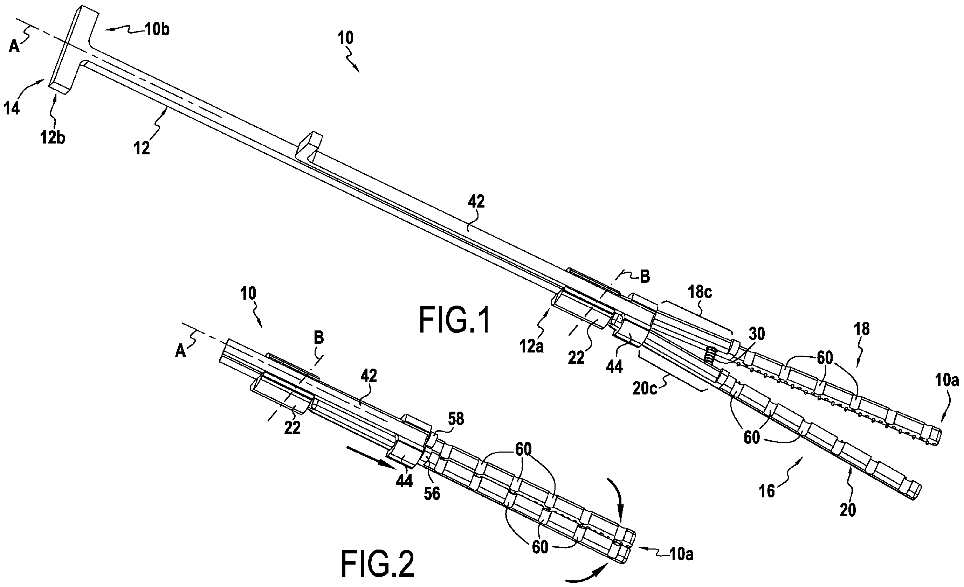

A l'aide des figures **1** à **3****,** on va tout d'abord décrire un instrument chirurgical **10** conforme à la présente invention. Cet instrument **10** est destiné à être utilisé pour modifier le volume de l'estomac d'un patient dans le cadre d'une opération de chirurgie bariatrique.

Comme on le constate sur la figure **1**, l'instrument **10** présente une forme longiligne et présente une extrémité distale **10a** et une extrémité proximale **10b.** Les expressions « distale » et « proximale » sont définies en référence à l'opérateur qui manipule l'instrument **10** selon l'invention.

L'instrument **10** comporte un corps **12** qui présente une extrémité distale **12a** et une extrémité proximale **12b**. On constate que l'extrémité proximale **10b** de l'instrument **10** correspond à l'extrémité proximale **12b** du corps.

En outre, le corps est muni, à son extrémité proximale **12b**, d'un dispositif de préhension **14** présentant la forme d'une poignée permettant au chirurgien de saisir fermement l'instrument **10**.

Dans cet exemple, le corps **12** présente la forme d'une tige s'étendant selon une direction longitudinale **A**. L'instrument est dimensionné de manière à pouvoir être introduit dans le corps du patient par voie laparoscopique.

Lorsque ledit instrument est destiné à être réutilisé pour plusieurs interventions chirurgicales, l'instrument est préférentiellement, mais pas exclusivement, réalisé dans un alliage métallique. Il peut s'agir par exemple, d'un alliage ASI 302 , un ASI 303, un ASI 304, un ASI 410, un ASI 416, un ASI 420, ou l'ASI 440, où ASI correspond à la classification de l'American Iron and Steel Institute (A.S.I.).

Lorsque l'instrument doit être jeté après son utilisation, il peut être réalisé dans une matière plastique très rigide comme par exemple le polymère renforcé de référence IXEF1032 (marque déposée) de la société Solvay. Le IXEF1032 (marque déposée) est un polyarylamide renforcé de 60% de fibre de verre qui a un module de flexion élevé de l'ordre de 21GPa (réalisé selon l'ISO 178). Il peut aussi être réalisé avec le IXEF3008 (marque déposée) qui est un polyarylamide renforcé de 30% de fibre de carbone et qui possède un module de flexion de l'ordre de 23 GPa (réalisé selon l'ISO 178).

A l'extrémité opposée du dispositif de préhension **14**, l'instrument **10** comporte un dispositif de serrage **16** qui comporte un premier bras **18** et un second bras **20** montés pivotants l'un par rapport à l'autre à l'extrémité distale **12a** du corps **12**.

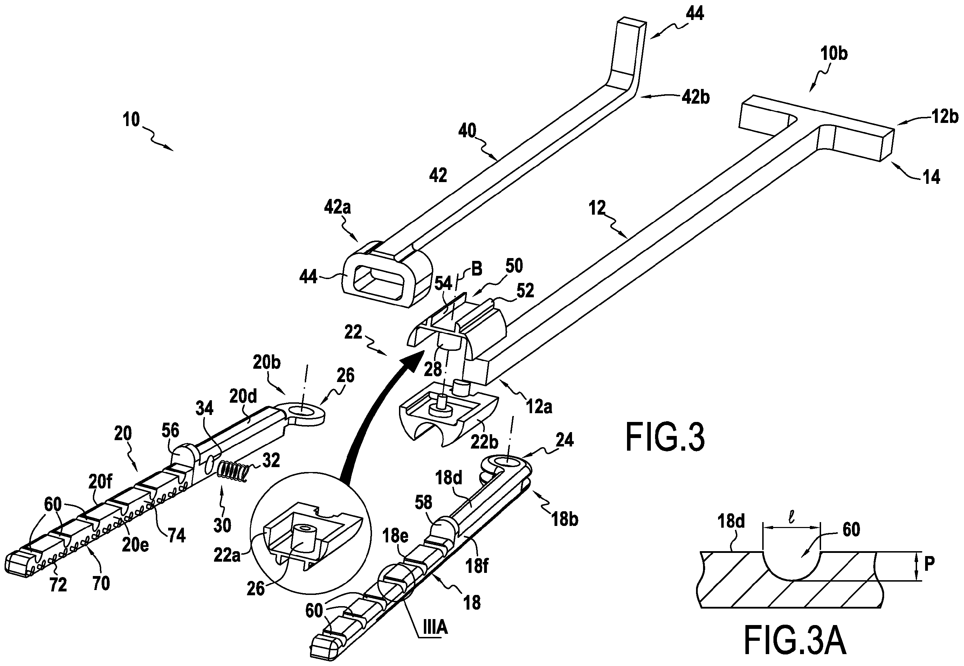

Comme on le constate sur la figure **3**, chacun des premier et second bras **18**, **20** présente une extrémité proximale **18b**, **20b** reliée de manière pivotante à l'extrémité distale **12a** du corps **12**. Plus précisément les extrémités **18b**, **20b** des premier et second bras **18**, **20** qui sont reliées de manière pivotante à l'extrémité distale du corps **12** sont montées pivotantes autour d'un même axe de rotation **B**. Le dispositif de serrage **16**, comportant les premier et second bras **18**, **20**, forme donc une pince.

Pour réaliser la liaison pivot (autour de l'axe de rotation **B**) des premier et second bras **18**, **20** par rapport au corps **12**, l'instrument **10** comporte dans cet exemple une pièce de liaison **22** qui est fixée solidairement à l'extrémité distale **12a** du corps **12**. Dans l'exemple de la figure **3**, la pièce de liaison **22** est constituée de deux pièces **22a, 22b** fixées l'une à l'autre tout en enserrant l'extrémité distale **12a** du corps **12**. Les extrémités proximales **18b**, **20b** des premier et second bras **18**, **20** comportent des oeillets **24**, **26** qui sont coaxiaux et qui coopèrent avec un axe de pivot **26** appartenant à la pièce de liaison **22**.

L'instrument **10** comporte en outre un dispositif d'ouverture **30** qui a pour fonction d'écarter les premier et second bras **18**, **20** l'un de l'autre en les faisant pivoter l'un par rapport à l'autre autour de l'axe **B**. Dans cet exemple, le dispositif d'ouverture **30** comporte un organe ressort **32** qui relie les premier et second bras **18**, **20** en tendant à les maintenir écartés l'un de l'autre. Comme on le voit sur la figure **3**, l'organe ressort **32** est, dans cet exemple, un ressort cylindrique qui s'étend entre les deux bras tout en étant logé dans des cavités **34** qui sont ménagées dans les faces latérales intérieures **18e**, **20e** des bras **18**, **20**.

L'instrument **10** comporte en outre un dispositif de fermeture **40** qui a pour fonction de rapprocher les premier et second bras **18**, **20** l'un de l'autre en les faisant pivoter l'un par rapport à l'autre autour de l'axe **B**.

Dans cet exemple, le dispositif de fermeture **40** comporte un bras d'actionnement **42** présentant une extrémité distale **42a** et une extrémité proximale **42b**. Le bras d'actionnement **42** se présente sous la forme d'une tige dont l'extrémité proximale **42b** comprend un organe de préhension **44** sous forme d'un renvoi orthogonal. La tige **42** s'étend selon la direction longitudinale **A** du corps **12**.

Le dispositif de fermeture comprend un organe de fermeture 44 fixé à l'extrémité distale **42a** du bras d'actionnement **42**. Cet organe de fermeture **44** se présente sous la forme d'un manchon. Comme on le constate sur les figures **1** et **2**, l'organe de fermeture **44** en forme de manchon est configuré pour enserrer l'ensemble constitué des premier et second bras **18**, **20**, tout en permettant le coulissement des bras dans le manchon. Ainsi, l'actionnement de l'organe de fermeture par le chirurgien entraîne le rapprochement des premier et second bras l'un de l'autre.

Plus spécifiquement, l'organe de fermeture **44** en forme de manchon flanque les premier et second bras **18**, **20** de telle sorte que le déplacement du bras d'actionnement **42** vers l'extrémité distale **10a** de l'instrument **10** entraîne le rapprochement des premier et second bras **18**, **20** l'un de l'autre. En d'autres termes, comme cela est illustré sur la figure **2**, l'actionnement du dispositif de fermeture, par un déplacement du bras d'actionnement vers l'extrémité distale **10a** de l'instrument conduit à amener l'instrument **10** vers sa position fermée.

On comprend donc que l'organe de fermeture **44** est monté coulissant le long des portions **18c**, **20c** des premier et second bras **18**, **20**, qui sont disposées à proximité de l'extrémité distale **12a** du corps **12**.

Pour ce faire, l'organe de fermeture **44** est monté sur le corps **12** de manière coulissante parallèlement à la direction longitudinale **A** dudit corps.

Ce montage coulissant est obtenu grâce au fait que le bras d'actionnement **42** coopère avec un dispositif de guidage **50** qui est monté sur le corps afin de permettre au bras d'actionnement de pouvoir coulisser selon la direction longitudinale **A** du corps. Dans cet exemple, le dispositif de guidage **50** est constitué de deux nervures **52**, **54** s'étendant parallèlement à la direction longitudinale du corps tout en faisant saillie depuis la portion supérieure **22a** de la pièce de liaison **22**. Le dispositif de guidage **50** forme ainsi une glissière dans laquelle coulisse le bras d'actionnement **42**.

En se référant à la figure **3**, on constate que chacun des premier et second bras **18**, **20** comporte en outre un organe de butée **56**, **58** configuré pour limiter le déplacement de l'organe de fermeture **44** vers l'extrémité distale **10a** de l'instrument **10**. Ces organes de butée **56**, **58** font saillie depuis les faces latérales supérieures **18d**, **20d** des premier et second bras **18**, **20.**

En se référant à nouveau aux figures **1** et **2**, on conçoit que l'amplitude de déplacement du bras d'actionnement **42** est comprise entre la pièce de liaison **22** et les organes de butée **56**, **58**.

Compte-tenu de ce qui précède, on comprend que l'instrument **10** présente une position ouverte, illustrée sur la figure **1**, dans laquelle les premier et second bras **18**, **20** sont écartés l'un de l'autre grâce à l'organe ressort **32**, et une position fermée, illustrée sur la figure **2**, dans laquelle les premier et second bras **18**, **20** sont positionnés l'un contre l'autre grâce à l'organe de fermeture **44**. Lors de l'avancée de l'organe de fermeture vers l'extrémité distale **10a** de l'instrument **10**, les bras sont rapprochés l'un de l'autre en allant à l'encontre de la force exercée par l'organe ressort **32**.

En se référant à la figure **2**, on constate par ailleurs que le dispositif de fermeture **40** présente une position verrouillée dans laquelle l'instrument **10** est maintenu en position fermée. Dans cette position verrouillée, les bras ne peuvent pas s'écarter l'un de l'autre.

Lorsque l'organe de fermeture **44** est déplacé vers l'extrémité proximale **10b** de l'instrument **10** de manière à venir au contact de la pièce de liaison **22**, l'organe de fermeture **44** libère le pivotement des premier et second bras **18**, **20**, en sorte que l'organe ressort écarte les bras l'un de l'autre. En d'autres termes, dans cette position déverrouillée du dispositif de fermeture, l'instrument est libre d'être amené en position ouverte grâce au dispositif d'ouverture **30**.

En se référant de nouveau à la figure **3**, on constate que chacun des premier et second bras **18**, **20** comporte une pluralité de guides **60**. Pour chacun des bras, ces guides présentent la forme d'encoches qui débouchent dans la face supérieure **18d**, **20d** des bras ainsi que dans les faces latérales intérieures **18e**, **20e** et extérieures **18f**, **20f** des bras. Ces encoches **60** sont disposées à intervalles réguliers entre les organes de butée et les extrémités distales des bras. Dans cet exemple, chacun des bras comporte six encoches.

Comme on le verra ci-dessous, ces guides en forme d'encoches sont agencés pour permettre le passage d'une aiguille à suture.

En se référant à la figure **2**, on constate que les encoches **60** des premier et second bras **18**, **20** sont disposées en vis-à-vis de manière à former un passage transversal continu pour l'aiguille. Dans cet exemple la longueur des encoches correspond sensiblement à la largeur des bras, tandis que la profondeur **P** de l'encoche est de l'ordre de 5 millimètres, la largeur **I** de l'encoche étant de l'ordre de 3 millimètres.

En se référant à la figure **3A**, on constate que la section transversale des encoches présente une forme divergente s'ouvrant vers les faces supérieures **18d**, **20d** des bras. L'intérêt de cette forme, en l'espèce convexe permet un retrait aisé des fils hors des encoches lors du retrait de l'instrument.

Selon un autre aspect de l'invention, chacun des premier et second bras comporte une portion d'accroche **70** s'étendant selon la face latérale intérieure **18e**, **20e** des bras. Dans cet exemple, la portion d'accroche **70** comprend une pluralité de picots **72** qui s'étendent le long des faces latérales intérieures des premier et second bras. Ces picots **72** font saillie depuis les faces latérales intérieures **18e**, **20e** des bras, de manière à venir s'accrocher avec les tissus. Les bras comportent par ailleurs des orifices **74** qui s'étendent également selon des faces latérales intérieures des bras, ces orifices **74** étant destinés à recevoir les picots **72** lorsque les premier et second bras sont amenés l'un contre l'autre en position fermée de l'instrument **10**.

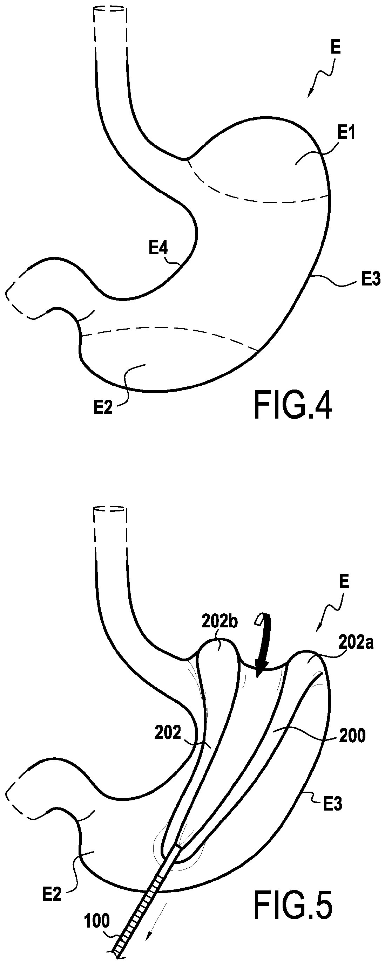

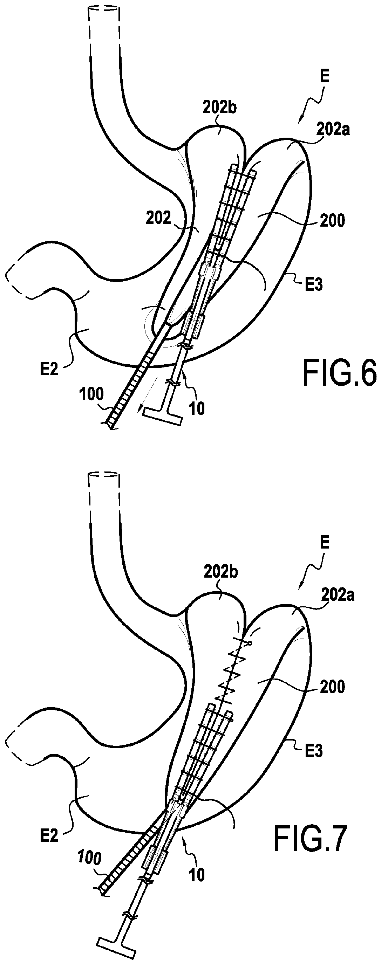

A l'aide des figures **4** à **7**, on va maintenant décrire un exemple de mise en oeuvre d'un procédé de modification du volume de l'estomac **E** d'un patient. De manière classique l'estomac **E** comporte une partie supérieure **E1** généralement appelée fundus. La partie inférieure de l'estomac **E2** est appelée fond de l'estomac **E**. L'estomac **E** présente par ailleurs une grande courbure **E3** ainsi qu'une petite courbure **E4**, les petites et grandes courbures **E4**, **E3** étant disposées entre le fundus et le fond de l'estomac.

Selon une première étape, on saisit une portion du fundus **E1** de l'estomac, par exemple à l'aide d'une pince de célioscopie **100**. La portion du fundus qui a été saisie est amenée, à l'aide de la pince **100**, vers le fond de l'estomac ce qui a pour effet de former deux bourrelets **200**, **202** qui s'étendent sensiblement selon la grande courbure **E3** de l'estomac **E**. La réalisation de cette plicature est illustrée sur la figure **5**.

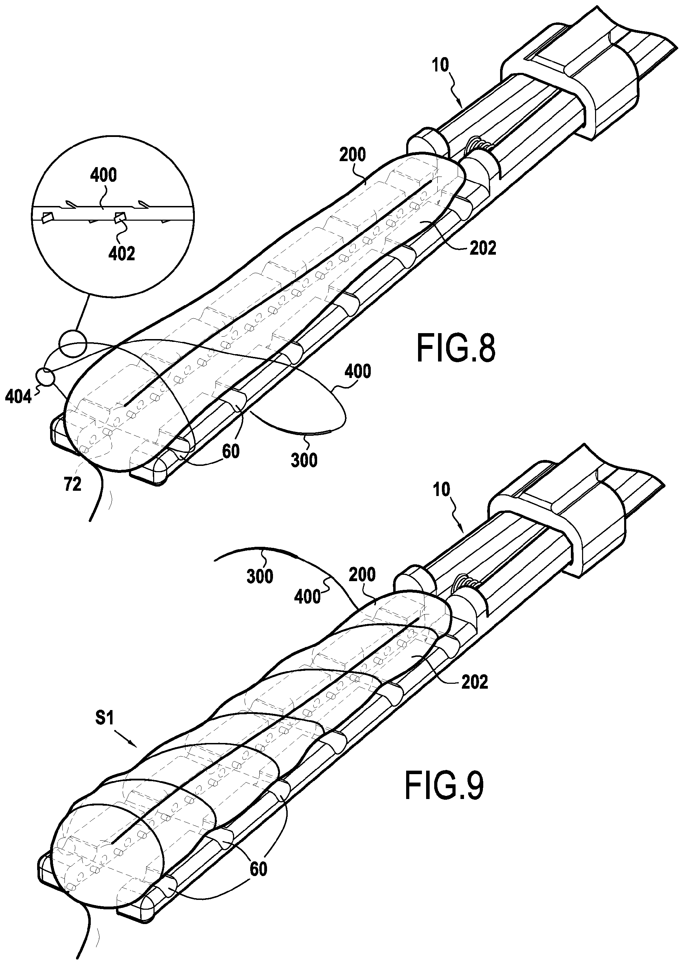

Ensuite, tout en maintenant la plicature à l'aide de la pince de célioscopie **100**, on rapproche les deux bourrelets **200**, **202** l'un de l'autre à l'aide de l'instrument **10** selon l'invention. Pour ce faire, on commence par approcher les parties supérieures **200a**, **200b** des bourrelets **200**, **202** qui sont disposés à proximité du fundus de l'estomac. L'instrument **10** est amené en position ouverte à proximité du fond de l'estomac, les premier et second bras **18**, **20** étant disposés de part et d'autre des bourrelets **200** et **202.** Puis on actionne le dispositif de fermeture de manière à rapprocher les premier et second bras l'un de l'autre, ce qui a pour effet d'amener l'instrument en position fermée. Puis on réalise un premier surjet **S1** à l'aide d'une aiguille à suture **300** et un fil de suture **302**. Le surjet est réalisé en faisant passer successivement l'aiguille **300** dans les encoches **60** des premier et second bras de manière à traverser les deux bourrelets ; ainsi que dans une boucle d'extrémité **404** du fil **400** jusqu'à former le premier surjet **S1,** comme illustré sur la figure **9**.

Le fil **400** est cranté. Il comprend sur sa surface extérieure des harpons **402** qui permettent avantageusement de maintenir le fil de suture serré.

Après que le premier surjet **S1** a été réalisé, l'instrument **10** est déplacé vers le fond de l'estomac puis actionné de manière à rapprocher les parties médianes des deux bourrelets **200**, **202** l'une de l'autre. On réalise ensuite un deuxième surjet **S2.** Puis l'instrument **10** est à nouveau déplacé et actionné de manière à rapprocher les parties supérieures des bourrelets **200**, **202** à la suite de quoi on réalise un troisième surjet **S3.**

Bien évidemment, sans sortir du cadre cet exemple on pourrait réaliser un nombre de surjet supérieur à trois.

Après que les bourrelets ont été suturés grâce aux trois surjets, l'instrument **10** et la pince coelioscopie sont retirés. La mise en oeuvre de ce procédé de modification du volume de l'estomac peut être réalisée très rapidement, en moins d'une heure par un chirurgien expérimenté.

Par ailleurs, les figures **8** à **9** illustrent un système **S** destiné à être utilisé pour modifier le volume de l'estomac **E** d'un patient, comportant un instrument **10** selon l'invention, une aiguille à suture **300** et au moins un fil de suture cranté **400** dont les crans forment des harpons.

## Revendications

1. Instrument (10) destiné à être utilisé pour modifier le volume de l'estomac d'un patient, ledit instrument présentant une extrémité distale (10a) et comprenant
un corps (12) ayant une extrémité distale (12a);
un dispositif de serrage (16) comportant des premier (18) et second (20) bras montés pivotants à l'extrémité distale (12a) du corps (12) tout en étant pivotants l'un par rapport à l'autre, chacun des premier et second bras (18,20) comportant une pluralité de guides pour le passage d'une aiguille à suture ;
un dispositif d'ouverture (30) pour écarter les premier et second bras (18,20) l'un de l'autre en les faisant pivoter l'un par rapport à l'autre, ledit dispositif d'ouverture comportant un organe ressort (32) reliant les premier et second bras (18,20) et tendant à les maintenir écartés l'un de l'autre ; et
un dispositif de fermeture (40) pour rapprocher les premier et second bras (18,20) l'un de l'autre en les faisant pivoter l'un par rapport à l'autre.

2. Instrument selon la revendication 1, présentant une position ouverte dans laquelle les premier et second bras (18,20) sont écartés l'un de l'autre grâce au dispositif d'ouverture (30), et une position fermée dans laquelle les premier et second bras (18,20) sont amenés l'un vers l'autre grâce au dispositif de fermeture (40), le dispositif de fermeture (40) présentant une position verrouillée dans laquelle l'instrument (10) est maintenu en position fermée, et une position déverrouillée dans laquelle l'instrument est libre d'être amené en position ouverte par le dispositif d'ouverture (30).

3. Instrument selon l'une quelconque des revendications 1 à 2, dans lequel chacun des premier (18) et second (20) bras présente une extrémité (18b,20b) reliée de manière pivotante à l'extrémité distale du corps.

4. Instrument selon la revendication 3, dans lequel les extrémités (18b,20b) des premier et second bras (18,20) reliées de manière pivotante à l'extrémité distale (12a) du corps (12) sont montées pivotantes autour d'un même axe de rotation (13).

5. Instrument selon l'une quelconque des revendications 1 à 4, dans lequel le dispositif de fermeture (40) comporte un organe de fermeture (44) actionnable coopérant avec les premier et second bras (18,20) de sorte que l'actionnement de l'organe de fermeture par le chirurgien entraine le rapprochement des premier et second bras l'un de l'autre.

6. Instrument selon la revendication 5, dans lequel le corps (12) présente une direction longitudinale (A), et dans lequel l'organe de fermeture (44) est monté sur le corps (12) de manière coulissante parallèlement à ladite direction longitudinale.

7. Instrument selon la revendication 6, dans lequel le dispositif de fermeture comporte un bras d'actionnement (42) qui coopère avec un dispositif de guidage (56) monté sur le corps permettant au bras d'actionnement de coulisser selon la direction longitudinale (A) du corps, dans lequel l'organe de fermeture (44) est disposé à une extrémité distale (42a) du bras d'actionnement (42) et flanque les premier et second bras (18,20) de telle sorte que le déplacement du bras d'actionnement (42) vers l'extrémité distale (10a) de l'instrument (10) entraîne le rapprochement des premier et second bras l'un de l'autre.

8. Instrument selon la revendication 6 ou 7, comportant en outre au moins un organe de butée (56, 58) configuré pour limiter le déplacement de l'organe de fermeture (44) vers l'extrémité distale (10a) de l'instrument (10).

9. Instrument selon la revendication 8, dans lequel l'organe de butée (56,58) fait saillie depuis l'un des premier et second bras (18,20).

10. Instrument selon l'une quelconque des revendications 1 à 9, dans lequel au moins le premier bras (18) comporte une portion d'accroche (70) s'étendant selon une face latérale intérieure (18e) du premier bras disposée en vis-à-vis d'une face latérale intérieure (20e) du second bras (20).

11. Instrument selon la revendication 10, dans lequel la portion d'accroche comprend une pluralité de picots (72) s'étendant le long de la face latérale intérieure du premier bras.

12. Instrument selon la revendication 11, dans lequel au moins le second bras (20) comporte des orifices (74) s'étendant selon la face latérale intérieure du second bras en vis-à-vis de la face latérale intérieure du premier bras, lesdits orifices (74) étant destinés à recevoir les picots (72) du premier bras lorsque les premier et second bras sont amenés l'un contre l'autre.

13. Instrument selon l'une quelconque des revendications 1 à 12, dans lequel les guides (60) débouchent transversalement de part et d'autre de chacun des premier et second bras.

14. Instrument selon la revendication 13, dans lequel les premier et second bras (18,20) présentent des faces supérieures (18d,20d), et dans lequel les guides sont des encoches débouchant dans les faces supérieures des premier et second bras, et dans lequel la section transversale des encoches (60) présente de préférence une forme divergente s'ouvrant vers les faces supérieures (18d,20d).

15. Système (S) destiné à être utilisé pour modifier le volume de l'estomac (E) d'un patient, comportant un instrument (10) selon l'une quelconque des revendications 1 à 14, une aiguille à suture (300), et au moins un fil de suture cranté (400) dont les crans forment des harpons.

## Patentansprüche

1. Instrument, das dazu bestimmt ist, zur Modifizierung des Volumens des Magens eines Patienten verwendet zu werden, wobei das Instrument ein distales Ende (10a) besitzt und aufweist
einen Körper (12), der ein distales Ende (12a) aufweist,
eine Spannvorrichtung (16), umfassend einen ersten (18) und einen zweiten (20) Arm, die schwenkbar an dem distalen Ende (12a) des Körpers (12) befestigt sind und dabei gleichzeitig relativ zueinander schwenkbar sind, wobei jeder des ersten und zweiten Arms (18, 20) mehrere Führungen für den Durchgang einer Suturnadel aufweist,
eine Öffnungsvorrichtung (30), um den ersten und zweiten Arm (18, 20) voneinander zu spreizen, indem sie relativ zueinander verschwenkt werden, wobei die Öffnungsvorrichtung ein Federorgan (32) aufweist, das den ersten und zweiten Arm (18, 20) verbindet und dazu neigt, sie voneinander beabstandet zu halten, und
eine Schließvorrichtung (40), um den ersten und zweiten Arm (18, 20) aneinander anzunähern, indem sie relativ zueinander verschwenkt werden.

2. Instrument nach Anspruch 1, aufweisend
eine geöffnete Position, in der der erste und zweite Arm (18, 20) dank der Öffnungsvorrichtung (30) voneinander beabstandet sind, und eine geschlossene Position, in der der erste und zweite Arm (18, 20) dank der Schließvorrichtung (40) zueinander geführt werden, wobei die Schließvorrichtung (40) aufweist
eine Verriegelungsposition, in der das Instrument (10) in geschlossener Position gehalten wird, und eine Entriegelungsposition, in der das Instrument frei ist, um von der Öffnungsvorrichtung (30) in die geöffnete Position gebracht zu werden.

3. Instrument nach einem der Ansprüche 1 bis 2, wobei der erste (18) und der zweite (20) Arm jeweils ein Ende (18b, 20b) aufweisen, das schwenkbar mit dem distalen Ende des Körpers verbunden ist.

4. Instrument nach Anspruch 3, wobei die Enden (18b, 20b) des ersten und des zweiten Arms (18, 20), die schwenkbar mit dem distalen Ende (12a) des Körpers (12) verbunden sind, schwenkbar um eine gleiche Drehachse (13) befestigt sind.

5. Instrument nach einem der Ansprüche 1 bis 4, wobei die Schließvorrichtung (40) ein betätigbares Schließorgan (44) aufweist, das mit dem ersten und dem zweiten Arm (18, 20) derart zusammenwirkt, dass das Betätigen des Schließorgans durch den Chirurgen das Annähern des ersten und zweiten Arms aneinander herbeiführt.

6. Instrument nach Anspruch 5, wobei der Körper (12) eine Längsrichtung (A) aufweist und wobei das Schließorgan (44) parallel verschiebbar zu der Längsrichtung auf dem Körper (12) befestigt ist.

7. Instrument nach Anspruch 6, wobei die Schließvorrichtung einen Betätigungsarm (42) aufweist, der mit einer Führungsvorrichtung (56) zusammenwirkt, die auf dem Körper befestigt ist, die dem Betätigungsarm ermöglicht, in der Längsrichtung (A) des Körpers verschoben zu werden, wobei das Schließorgan (44) an einem distalen Ende (42a) des Betätigungsarms (42) angeordnet ist und den ersten und zweiten Arm (18, 20) derart flankiert, dass das Verschieben des Betätigungsarms (42) zu dem distalen Ende (10a) des Instrumentes (10) das Annähern des ersten und zweiten Arms aneinander herbeiführt.

8. Instrument nach Anspruch 6 oder 7, ferner umfassend mindestens ein Anschlagorgan (56, 58), das dazu konfiguriert ist, das Verschieben des Schließorgans (44) zu dem distalen Ende (10a) des Instrumentes (10) zu begrenzen.

9. Instrument nach Anspruch 8, wobei das Anschlagorgan (56, 58) von dem ersten oder dem zweiten Arm (18, 20) vorsteht.

10. Instrument nach einem der Ansprüche 1 bis 9, wobei zumindest der erste Arm (18) einen Einhakabschnitt (70) aufweist, der sich entlang einer inneren Seitenfläche (18e) des ersten Arms erstreckt, die gegenüber einer inneren Seitenfläche (20e) des zweiten Arms (20) angeordnet ist.

11. Instrument nach Anspruch 10, wobei der Einhakabschnitt mehrere Stifte (72) aufweist, die sich entlang der inneren Seitenfläche des ersten Arms erstrecken.

12. Instrument nach Anspruch 11, wobei zumindest der zweite Arm (20) Öffnungen (74) aufweist, die sich entlang der inneren Seitenfläche des zweiten Arms gegenüber der inneren Seitenfläche des ersten Arms erstrecken, wobei die Öffnungen (74) dazu bestimmt sind, die Stifte (72) des ersten Arms aufzunehmen, wenn der erste und zweite Arm zueinander geführt werden.

13. Instrument nach einem der Ansprüche 1 bis 12, wobei die Führungen (60) in Längsrichtung beiderseits von jedem des ersten und des zweiten Arms münden.

14. Instrument nach Anspruch 13, wobei der erste und der zweite Arm (18, 20) obere Flächen (18d, 20d) aufweisen, und wobei die Führungen Kerben sind, die in die obere Flächen des ersten und des zweiten Arms münden, und wobei der Querschnitt der Kerben (60) vorzugsweise eine auseinandergehende Form aufweist, die sich in Richtung der oberen Flächen (18d, 20d) öffnet.

15. System (S), das dazu bestimmt ist, verwendet zu werden, um das Volumen des Magens (E) eines Patienten zu modifizieren, umfassend ein Instrument (10) nach einem der Ansprüche 1 bis 14, eine Suturnadel (300) und mindestens einen gekerbten Suturfaden (400), dessen Kerben Harpunen bilden.

## Claims

1. An instrument (10) intended to be used to modify the volume of the stomach of a patient, said instrument having a distal end (10a) and comprising:
a body (12) having a distal end (12a);
a clamping device (16) comprising first (18) and second (20) arms pivotably mounted at the distal end (12a) of the body (12) while pivoting relative to each other, each of the first and second arms (18, 20) comprising a plurality of guides for passage of a suture needle;
an opening device (30) for moving the first and second arms (18, 20) apart from each other by having them pivot relative to each other, said opening device comprising a spring member (32) connecting the first and second arms (18, 20) and tending to keep them apart from each other; and
a closing device (40) for moving the first and second arms (18, 20) towards each other by having them pivot relative to each other.

2. The instrument according to claim 1, having an open position wherein the first and second arms (18, 20) are moved apart from each other because of the opening device (30), and a closed position wherein the first and second arms (18, 20) are brought towards each other because of the closing device (40), and the closing device (40) having a locked position wherein the instrument (10) is kept in a closed position, and an unlocked position wherein the instrument is free to be guided to the open position by the opening device (30).

3. The instrument according to any one of claims 1 to 2, wherein each of the first (18) and second (20) arm has an end (18b, 20b) pivotably attached at the distal end of the body.

4. The instrument according to claim 3, wherein the ends (18b, 20b) of the first and second arms (18, 20) pivotably attached at the distal end (12a) of the body (12) are pivotably mounted about the same axis of rotation (13).

5. The instrument according to any one of claims 1 to 4, wherein the closing device (40) comprises an actuatable closing member (44) cooperating with the first and second arms (18, 20) such that the actuation of the closing member by the surgeon causes the first and second arms to move towards each other.

6. The instrument according to claim 5, wherein the body (12) has a longitudinal direction (A), and wherein the closing member (44) is slidably mounted on the body (12) parallel to said longitudinal direction.

7. The instrument according to claim 6, wherein the closing device comprises an actuating arm (42) which cooperates with a guide device (56) mounted on the body allowing the actuating arm to slide along the longitudinal direction (A) of the body, wherein the closing member (44) is arranged at a distal end (42a) of the actuating arm (42) and flanks the first and second arms (18, 20) such that displacement of the actuating arm (42) towards the distal end (10a) of the instrument (10) causes the first and second arms to move towards each other.

8. The instrument according to claim 6 or 7, further comprising at least one stop member (56, 58) configured to limit displacement of the closing member (44) towards the distal end (10a) of the instrument (10).

9. The instrument according to claim 8, wherein the stop member (56, 58) projects from one of the first and second arms (18, 20).

10. The instrument according to any one of claims 1 to 9, wherein at least the first arm (18) comprises a hooking portion (70) extending according to an inner lateral face (18e) of the first arm arranged facing an inner lateral face (20e) of the second arm (20).

11. The instrument according to claim 10, wherein the hooking portion comprises a plurality of barbs (72) extending along the inner lateral face of the first arm.

12. The instrument according to claim 11, wherein at least the second arm (20) comprises orifices (74) extending along the inner lateral face of the second arm facing the inner lateral face of the first arm, said orifices (74) being intended to receive the barbs (72) of the first arm when the first and second arms are brought against each other.

13. The instrument according to any one of claims 1 to 12, wherein the guides (60) terminate transversally on either side of each of the first and second arms.

14. The instrument according to claim 13, wherein the first and second arms (18, 20) have upper faces (18d, 20d), and wherein the guides are slots terminating in the upper faces of the first and second arms, and wherein the cross-section of the slots (60) has a divergent shape opening towards the upper faces (18d, 20d).

15. A system (S) intended to be used to modify the volume of the stomach (E) of a patient, comprising an instrument (10) according to any one of claims 1 to 14, a suture needle (300), and at least one notched suture thread (400) whereof the notches form harpoons.
